# Europäisches Patentamt
## European Patent Office
## Office européen des brevets

(11) Veröffentlichungsnummer: **0 158 235**
**B1**

(12) # EUROPÄISCHE PATENTSCHRIFT

(45) Veröffentlichungstag der Patentschrift:
**16.09.87**

(51) Int. Cl.⁴: **C 07 C  139/14**

(21) Anmeldenummer: **85103732.5**

(22) Anmeldetag: **28.03.85**

(54) **Verfahren zur Abtrennung der restlichen Schwefelsäure aus dem bei der Sulfoxidation von Paraffinen anfallenden Reaktionsgemisch.**

(30) Priorität: **05.04.84 DE 3412844**

(43) Veröffentlichungstag der Anmeldung:
**16.10.85 Patentblatt 85/42**

(45) Bekanntmachung des Hinweises auf die Patenterteilung:
**16.09.87 Patentblatt 87/38**

(84) Benannte Vertragsstaaten:
**DE FR IT NL**

(56) Entgegenhaltungen:
**DE - A - 2 855 849**

(73) Patentinhaber: **HOECHST AKTIENGESELLSCHAFT,
Postfach 80 03 20, D-6230 Frankfurt am Main 80 (DE)**

(72) Erfinder: **Höroldt, Ernst, Dr., Theresenstrasse 33,
D-6233 Kelkheim (Taunus) (DE)**
Erfinder: **Urschel, Adam, Dr., Gagernring 55,
D-6233 Kelkheim (Taunus) (DE)**

## Beschreibung

Bei der Sulfoxidation von Paraffinen (n-Alkanen) mit Schwefelfioxid, Sauerstoff und Wasser in Gegenwart von UV-Licht nach dem bekannten Licht-Wasser-Verfahren entsteht neben den gewünschten Paraffinsulfonsäuren auch Schwefelsäuse (Chemie in unserer Zeit, 13 [1979], Seite 157 ff). Im Verlauf der weiteren Aufarbeitung muss nun die Schwefelsäure aus der von der Hauptmenge des nicht umgesetzten Paraffins befreiten und entgasten Produktphase entfernt werden. Diese Abtrennung der Schwefelsäure kann erfolgen, indem man die Produktphase direkt neutralisiert beispielsweise mit Natronlauge und das anfallende Natriumsulfat abtrennt. Diese Methode ist jedoch nachteilig, da hier die gesamte vorhandene Schwefelsäure neutralisiert wird, was einen hohen Verbrauch an Alkali bedeutet, und man ausserdem ein Produkt erhält, das sich nicht weiterverwenden lässt, nämlich Natriumsulfat.

Man ist deshalb bemüht, aus der Produktphase zunächst durch andere Operationen einen möglichst grossen Teil der freien Schwefelsäure durch Phasentrennung abzuscheiden. Dies kann geschehen durch Zugabe von geeigneten Lösemitteln zur Produktphase, wobei man eine Entmischung erreicht in eine die Alkansulfonsäure enthaltende organische Phase und eine wässrige Phase, die aus 15-25%iger Schwefelsäure besteht. Eine Zusammenstellung derartiger Verfahren findet sich in der deutschen Patentschrift 3 013 808. Dabei kann aber die Schwefelsäure nicht quantitativ entfernt werden und der Rest verbleibt nach der Neutralisation als Natriumsulfat im Endprodukt. Ausserdem haftet allen Lösemittelverfahren der Nachteil an, dass eine zusätzliche Abtrennung und Aufarbeitung des Lösemittels bzw. des Lösemittelgemischs erforderlich ist.

Nach dem in der DE-OS 3 048 058 und DE-OS 3 210 573 beschriebenen Verfahren kann die Schwefelsäure auch mit Hilfe organischer Salze, z.B. Tributylammoniumhxdrogensulfat oder anderer Amine aus der Produktphase als 20%ige Schwefelsäure abgeschieden werden. Die Herstellung aminfreier Alkansulfonsäuren nach diesem Verfahren gestaltet sich jedoch problemastisch.

Ebenfalls bekannt ist aus der DE-OS 2 855 849 ein Verfahren, die Schwefelsäure aus der Produktphase durch Teilneutralisation mit Natronlauge zum Bisulfat umzusetzen. Durch Abdestillieren von Wasser, gegebenenfalls unter Zusatz eines Lösemittels als Azeotrop, bis zur Stufe des Bisulfat-Monohydrats erreicht man eine Entmischung. Das Monohydrat ist bei 58°C flüssig, so dass man theoretisch bei 60°C das Salz als Unterphase ablassen kann. Die Praxis hat aber gezeigt, dass eine genaue Einstellung auf diesen Punkt unter Betriebsbedingungen nicht zu erreichen ist. Auch durch die Zugabe eines Salzes z.B. Natriumsulfat zur Produktphase kann man bei 95°C die Bildung einer zweiten Phase, bestehend aus Wasser, Salz und freie Säure erzwingen. Um jedoch zu einem brauchbaren, niedrigen Schwefelsäure-Restgehalt zu gelangen, muss man soviel Salz zusetzen, wie gewichtsmässig an Alkansulfonsäure vorhanden ist.

Bei dem Sulfoxidations-Verfahren, wie es im grosstechnischen Massstab ausgeübt wird (Chemie in unserer Zeit, 13 [1979], Seite 161), wird die Produktphase ohne Zusatz von Lösemitteln, im Vakuum konzentriert, wobei Wasser mit einem Teil des Paraffins abdestilliert. Beim Konzentrieren trennt sich der Destillationsrückstand in 50-65%ige wässrige Schwefelsäure und in ein Konzentrat der Zusammensetzung: 35,0-40,0% Alkansulfonsäure, 54,4-56,6% Paraffin, 4,0-5,5% Wasser und 1,6-3,1% Schwefelsäure.

Dieses Konzentrat wird mit Natronlauge neutralisiert und dann das restliche Paraffin mit Wasserdampf abgetrieben. Die zurückbleibende Alkansulfonat-Schmelze wird zu Schuppen oder wässrigen Lösungen konfektioniert. Die so gewonnen Alkansulfonate enthalten bei einer Konzentration von 60% Wirksubstrat maximal 4% Natriumsulfat. Während im allgemeinen dieser geringe Salzanteil nicht stört, kann er sich jedoch bei gewissen Spezialitäten oder bei Einstellungen mit höherer Konzentration nachteilig auswirken, da mit steigendem Natriumsulfatgehalt die Viskosität des Alkansulfonates zunimmt, so dass die Pumpfähigkeit nicht mehr gewährleistet ist und bei höherem Sulfatanteil immer mit einer Entmischung des Produktes durch Auskristallisieren von Salz gerechnet werden muss.

Aufgabe der vorliegenden Erfindung war es daher, den Schwefelsäure-Restgehalt in den nach diesem Verfahren anfallenden Konzentraten zu senken und so nahezu salzfreie Alkansulfonate herzustellen. Dabei sollten wesentliche Prozessschritte des ausgezeichneten und bewährten Produktionsverfahrens nicht verändert werden.

Gegenstand der Erfindung ist ein Verfahren zur Abtrennung der restlichen Schwefelsäure aus dem bei der Umsetzung von Paraffin mit Schwefeldioxid, Sauerstoff und Wasser unter Einstrahlung von UV-Licht erhaltenen Reaktionsgemisch, das durch Entgasung bei erhöhter Temperatur von Schwefeldioxid befreit wurde und aus dem durch Konzentrieren die Hauptmenge an Schwefelsäure abgetrennt wurde, indem man diese konzentrierte Produktphase mit mindestens sowiel eines Alkalicarbonats, -peroxids, -sulfats oder -sulfonats versetzt wie notwendig ist, um die restliche Schwefelsäure in Alkalibisulfat zu überführen.

Ausgangspunkt für das erfindungsgemässe Verfahren ist das sogenannte Konzentrat wie oben beschrieben. Zu diesem Konzentrat gibt man bei einer Temperatur von 20 bis 120, vorzugsweise 75 bis 100°C ein Alkalisalz der oben beschriebenen Art und zwar in einer Menge, die man aufgrund der stöchiometrischen Bedingungen benötigt, um die gesamte noch vorhandene Schwefelsäure in Alkalibisulfat zu überführen. Als derartige Salze kommen insbesondere die Natriumsalze in Frage wie Soda, Natriumperoxid, Natriumhydroxid, Natriumsulfat und auch die Natriumalkansulfonate selbst.

Vorzugsweise werden diese Salze wasserfrei eingesetzt, um die Bildung von schleimigem, schlecht abtrennbarem Bisulfat-Monohydrat zur vermeiden. Nimmt man wässrige Lösungen der Salze wird die Ausbeute an Kristallisat und die Kristallisationsgeschwindigkeit ungünstig beeinflusst. So kristallisiert bei Verwendung von nur 30%iger Natronlauge nahezu kein Salz mehr aus. Auch die Umsetzung mit Glaubersalz verläuft nicht optimal. Die Zugabe der Salze erfolgt portionsweise oder auch kontinuierlich in einer der Umsetzung angepassten Geschwindigkeit, um das Auftreten eines Alkaliüberschusses zu verhindern, der zur Bildung des nicht erwünschten Natriumsulfats oder zur Teilneutralisation von Alkansulfonsäure führen kann. Die Temperatur, bei der die Salze zu dem Konzentrat zugegeben werden, hängt im wesentlichen ab von der Löslichkeit dieser Salze. Im allgemeinen wird man bei den Temperaturen arbeiten, die das Konzentrat bereits aus der vorherigen Aufarbeitungsstufe hat, d.h. bei ca. 90-95°C. Die Verwendung von verdünnten wässrigen oder auch wässrig/alkoholischen oder mit anderen Lösemitteln verdünnten Alkali-Lösungen ist nach dem erfindungsgemässen Verfahren ebenfalls möglich. In diesem Fall muss dann das die Kristallisation des Bisulfats störende Wasser oder Lösemittel aus dem Reaktionsgemisch abgetrennt werden, vorzugsweise durch Abdestillieren im Vakuum.

Auch die Verwendung eines geringen Alkaliüberschusses zur Teilneutralisation, z.B. die 1,5fache stöchiometrische Menge stört den weiteren Prozessablauf im allgemeinen nicht. Hierbei scheidet sich dann neben der Hauptmenge Bisulfat ein geringer Anteil Natriumsulfat ab.

Das Ende der Teilneutralisation bei Einsatz eines festen, wasserfreien Alkalisalzes zeigt sich im allgemeinen dadurch an, dass eine klare Lösung erhalten wird, aus der nach Abkühlen unter ca. 60°C das hydratfreie Bisulfat auskristallisiert. Die Abtrennung des Kristallisats erfolgt auf üblichem Weg, z.B. über eine Filterzentrifuge. Das vom Salz befreite Konzentrat hat einen Schwefelsäure-Restgehalt um 0,3%. Dieses Konzentrat wird dann wie üblich aufgearbeitet, d.h. es wird neutralisiert und mit überhitztem Wasserdampf wird das restliche Paraffin abgetrieben.

Prinzipiell ist es möglich, als Fällungsmittel auch andere alkalisch reagierende Verbindungen einzusetzen, die mit der Schwefelsäure im Konzentrat unlösliche Salze bilden, wie z.B. die Carbonate von Barium oder Calcium. Der Nachteil derartiger Produkte liegt jedoch darin, dass bei nicht quantitativer Umsetzung oder Abtrennung der gebildeten Sulfate störende Fremdionen im Konzentrat verbleiben. Da auch nach anderen bekannten Sulfoxidations-Prozessen hergestellte Alkansulfonsäuren im Endprodukt bis zu 10% Natriumsulfat enthalten können, kann das vorliegende Verfahren auch dort mit Vorteil zur Abtrennung der Schwefelsäure eingesetzt werden, vorausgesetzt, es liegt ein in der Zusammensetzung analoges Konzentrat vor.

Die Prozentangaben in den folgenden Beispielen sind Gewichtsprozente.

*Beispiel 1*

In einem 20-l-Glasgefäss wurden 13 530 g eines Sulfoxidations-Konzentrates, bestehend aus 38,0% Alkansulfonsäure, 2,2% Schwefelsäure, 4,9% Wasser und 55,0% Paraffin auf 95°C erwärmt. Bei dieser Temperatur wurden unter Rühren innerhalb von 10 Minuten 160,98 g Natriumcarbonat portionsweise eingetragen. Die nach der Umsetzung klare Lösung wurde noch 60 Minuten bei 95°C nachgerührt und dann auf Raumtemperatur abgekühlt. Das auskristallisierte Salz wurde über eine Filterzentrifuge, die mit einem Filtertuch der Porenweite 5-10 µm belegt ist, bei 4500 U/min abgetrennt. Die potentiometrische Titration des Filtrates mit einer 0,1 n Cyclohexylaminlösung ergab einen Gehalt von 38,0% Alkansulfonsäure und 0,50% Schwefelsäure.

*Beispiel 2*

Zu 842 g Sulfoxidations-Konzentrat der Zusammensetzung 39,7% Alkansulfonsäure, 1,84% Schwefelsäure, 5,19% Wasser und 53,3% Paraffin wurde bei 92°C unter Rühren innerhalb von 20 Minuten eine Lösung aus 10,92 g Kaliumcarbonat und 10,92 g Wasser zugetropft. Es wurde noch 30 Minuten bei dieser Temperatur nachgerührt und dann die klare Lösung in ein Kristallisationsgefäss abgelassen. Bei Raumtemperatur wurde über ein Schottglasfilter G2 der Porenweite 40-100 µm das Salz unter Wasserstrahlpumpenvakuum abgesaugt. Das Filtrat enthielt 40,8% Alkansulfonsäure und 0,3% Schwefelsäure. Es wurden 19,2 g Salz = 81,9 d.Th. isoliert. Das Salz hatte eine Reinheit von 91,8%.

*Beispiel 3*

Es wurden 709 g eines Sulfoxidations-Konzentrates aus 38,6% Alkansulfonsäure, 1,8% Schwefelsäure, 4,0 Wasser und 55,6% Paraffin bei 90°C unter Rühren im Zeitraum von 10 Minuten mit 5,16 g Natriumperoxid versetzt. Diese Reaktionstemperatur wurde noch 1 h beibehalten und nach dem Abkühlen wurde die Suspension wie im Beispiel 2 beschrieben aufgearbeitet. Im Filtrat wurden nach der Titration 38,0% Alkansulfonsäure und 0,7% Schwefelsäure gefunden.

*Beispiel 4*

824 g Sulfoxidations-Konzentrat der im Beispiel 1 angegebenen Zusammensetzung wurden unter Rühren bei 95°C mit 21,97 g Natriumsulfat (wasserfrei) innerhalb von 15 Minuten versetzt und 1 h auf dieser Temperatur gehalten. Unter Rühren wurde abgekühlt. Der feinkristalline Niederschlag konnte mit einem Schott-Glasfilter G2 abgetrennt werden. Das Filtrat enthielt 39,7% Alkansulfonsäure und 0,7% Schwefelsäure. An Salz waren 35,5 g (84,2%ig) angefallen, entsprechend 80,5% der theoretischen Menge.

*Beispiel 5*

Zu 893 g Sulfoxidations-Konzentrat der im Beispiel 1 angegebenen Zusammensetzung wurden unter Rühren bei 92°C 49,8 g Natriumalkansulfonat (Mg 275,5) entsprechend seiner Löslichkeit eingetragen und die Reaktionsmischung dann 1 h weitergeheizt. Nach dem Abkühlen konnte das ausgefallene Salz, wie im Beispiel 2 beschrieben, abfiltriert werden. Die Titration des Filtrates ergab 42,5% Alkansulfonsäure und 0,8% Schwefelsäure.

*Beispiel 6*

Zu 854 g Sulfoxidations-Konzentrat der Zusammensetzung 35,9% Alkansulfonsäure, 2,5% Schwefelsäure, 5,5% Wasser und 56,1 Paraffin wurden unter Rühren bei 80°C innerhalb von 45 Minuten 66 g einer 8,5%igen Natronlauge zugetropft. Anschliessend wurden aus der Reaktionslösung unter einem Druck von 93 mbar und einer Temperatur von 80-85°C 84 g eines aus 90% Wasser bestehenden Wasser/Paraffin-Gemisches abdestilliert. Nach Abkühlen auf Raumtemperatur wurde aus dem Sumpf das kristallisierte Natriumbisulfat abfiltriert. Das Filtrat enthielt 36,4% Alkansulfonsäure und 0,7% Schwefelsäure. Es wurden 86% d.Th. Salz isoliert mit einer Reinheit von 90%.

## Patentansprüche

1. Verfahren zur Abtrennung der restlichen Schwefelsäure aus dem bei der Umsetzung von Paraffin mit Schwefeldioxid, Sauerstoff und Wasser unter Einstrahlung von ultraviolettem Licht erhaltenen Reaktionsgemisch, das durch Entgasung bei erhöhter Temperatur von Schwefeldioxid befreit wurde und aus dem durch Konzentration die Hauptmenge an Schwefelsäure abgetrennt wurde, dadurch gekennzeichnet, dass man die dabei erhaltene konzentrierte Produktphase mit soviel eines Alkalicarbonats, -peroxids, -hydroxids, -sulfats oder -sulfonats versetzt wie notwendig ist, um die restliche Schwefelsäure in Alkalibisulfat zu überführen.

2. Verfahren nach Anspurch 1, dadurch gekennzeichnet, dass man das Alkalisalz bei einer Temperatur von 20 bis 120°C zugibt.

3. Verfahren nach Anspruch 1, dadurch gekennzeichnet, dass man als Alkalisalz ein Natriumsalz nimmt.

4. Verfahren nach Anspruch 1, dadurch gekennzeichnet, dass man zur Bildung des Bisulfats eine wässrige Lösung des Alkalisalzes einsetzt und das die Kristallisation störende Wasser abdestilliert.

## Claims

1. A process for the removal of the residual sulfuric acid from the reaction mixture which has been obtained in the reaction of paraffin with sulfur dioxide, oxygen and water under irradiation with ultraviolet light and which has been freed from sulfur dioxide by degassing at an elevated temperature and from which the bulk of the sulfuric acid has been removed by concentration, which comprises adding to the concentrated product phase obtained in this way as much of an alkali metal carbonate, peroxide, hydroxide, sulfate or sulfonate as is necessary to convert the residual sulfuric acid into an alkali metal bisulfate.

2. The process as claimed in claim 1, wherein the alkali metal salt is added at a temperature of 20 to 120°C.

3. The process as claimed in claim 1, wherein the alcali metal salt taken is a sodium salt.

4. The process as claimed in claim 1, wherein an aqueous solution of the alkali metal salt is employed in order to form the bisulfate, and the water which interferes with the crystallization is removed by distillation.

## Revendications

1. Procédé pour séparer l'acide sulfurique résiduel du mélange réactionnel obtenu lors de la réaction de la praffine avec le dioxyde de soufre, l'oxygène et l'eau sous exposition à la lumière ultraviolette, le mélange ayant été débarrassé du dioxyde de soufre par dégazage à haute température et de la majeure partie de l'acide sulfurique par concentration, caractérisé en ce qu'on ajoute à la phase produit concentrée ainsi obtenue par un carbonate, un peroxyde, un hydroxyde, un sulfate ou un sulfonate de métal alcalin en une quantité suffisante pour convertir l'acide sulfurique résiduel en hydrogénosulfate de métal alcalin.

2. Procédé selon la revendication 1, caractérisé en ce que l'on ajoute le sel de métal alcalin à une temperature comprise entre 20 et 120°C.

3. Procédé selon la revendication 1, caractérisé en ce que le sel de métal alcalin est un sel de sodium.

4. Procédé selon la revendication 1, caractérisé en ce que, pour former l'hydrogénosulfate, on utilise une solution aqueuse du sel de métal alcalin et on élimine par distillation l'eau gênant la cristallisation.